# EUROPEAN PATENT APPLICATION

(11) **EP 3 949 960 A1**
(43) Date of publication of application: **09.02.2022**
(21) Application number: 20189916.8
(22) Date of filing: 06.08.2020
(51) Int. Cl.: A61K 31/365, A61K 9/00, A61P 25/00, A61P 25/02, A61P 27/00, A61P 43/00

(54) **COMPOUNDS (IN PARTICULAR SALOTENOLIDE) IN THE TREATMENT OF AXONAL DAMAGE**

(71) Applicant: Ruhr-Universität Bochum, 44801 Bochum (DE)
(72) Inventor: Fischer, Dietmar, 41541 Dormagen (DE)
(74) Representative: Michalski Hüttermann & Partner Patentanwälte mbB

(57) **Abstract**

The present invention relates to a compound according to formula (1) and particularly to salotenolide for use in the treatment of axonal damage.

## Description

Axonal damage following injuries or diseases of nerves that project into arms or legs often causes permanent functional loss. This is because nerves often do not regenerate completely. Also, diseases such as Guillain-Barre syndrome, facial palsy, and diabetic neuropathy may lead to axonal damage and permanent functional loss. Patients affected with such motor and sensitivity disorders suffer from a considerable burden, which is associated with severe impairments in the quality of life, both privately and professionally. The success of recovery sometimes depends on the rate of regeneration of the regrowing nerve fibers that re-innervate the target tissue. An essential goal of the research is, therefore, the development of new approaches to accelerate and facilitate nerve fiber growth.

Efforts were made to develop drugs that can accelerate axon regeneration and thereby improve functional recovery. WO 2016/169698 A1 discloses that the sesquiterpene lactones parthenolide and cnicin can accelerate nerve growth. Intraneural and intravenous injection of parthenolide and cnicin accelerated axonal regeneration and improved functional recovery in mice and rats *in vivo.* Both parthenolide and cnicin, however, only provide a small concentration range in which these compounds improve axonal regeneration, while higher concentrations show no or even detrimental effects.

Therefore, the object underlying the present invention was to provide compounds that promote axonal regeneration in a broader active concentration range compared to parthenolide and cnicin.

The problem is solved by a compound according to general formula (1) as given as follows: 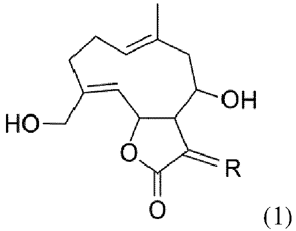 wherein:
- R: is selected from the group comprising -CH₂, -CHR', CR'₂, -NH and -NR",
- R': is selected from the group comprising C₁-C₁₀-alkyl, C₃-C₇-cycloalkyl, C₆-C₁₀-aryl, and C₇-C₁₀-arylalkyl; and
- R": is selected from the group comprising C₁-C₁₀-alkyl, C₃-C₇-cycloalkyl, C₆-C₁₀-aryl, and C₇-C₁₀-arylalkyl;
and/or racemates, enantiomers, diastereomers, solvates, hydrates, and pharmaceutically acceptable salts and/or esters thereof, with the proviso that the ester is not the 3-(3,4-dihydroxy-2-methylidene-butanoate), for use in the treatment of axonal damage.

Surprisingly, it was found that a compound of formula (1) markedly accelerated axon growth with a higher potency compared to cnicin and parthenolide. The compound accelerated axon growth of adult dorsal root ganglion neurons in culture with a fivefold higher potency compared to these compounds. A comparable promotion of nerve regeneration can thus be provided faster or at about a fifth of the concentration needed of cnicin or parthenolide. Consequently, the possible side effects of systemic drug administration can be reduced. The compound further provides a broader active concentration range and promoted axon growth of adult dorsal root ganglion neurons in culture in a fivefold broader concentration range compared to cnicin and parthenolide. Similarly, it could be further shown that salotenolide can also promote nerve regeneration of central neurons (retinal ganglion cells) with very high potency.

The term "alkyl" as used herein is to be understood as including linear and branched alkyl groups, if not defined otherwise. The term "C₁-C₁₀-alkyl" as used herein refers to linear or branched alkyl groups having 1 to 10 carbon atoms. Preferred are linear C₁-C₄-alkyl groups selected from the group comprising methyl, ethyl, n-propyl, and n-butyl. The term "aryl", as used herein, is to be understood as including aromatic groups having 6 to 10 carbon atoms. A preferred aryl group is phenyl. The term "arylalkyl" as used herein, is to be understood as being bound via the alkyl part. A preferred arylalkyl group is benzyl.

The group R may be selected from -NH or -NR". A group -NH or -NR" may be present when the compound is synthesized via a Michael addition. The group R" preferably is selected from methyl, ethyl, n-propyl, phenyl, or benzyl. The group R" may be selected from C₁-C₄-alkyl, preferably linear C₁-C₃-alkyl.

In embodiments, the group R is selected from -CH₂, -CHR', or CR'₂. The group R' preferably is selected from methyl, ethyl, n-propyl, phenyl, or benzyl. The group R' may be selected from C₁-C₄-alkyl, preferably linear C₁-C₃-alkyl. In preferred embodiments, the group R is-CH₂.

The compound comprises chiral centers, and thus, its racemates, enantiomers, or stereoisomers are possible and also usable. A preferred stereoisomer of the compound according to formula (1) is the compound according to formula (2) as indicated below and pharmaceutically acceptable salts or esters thereof, with the proviso that the ester is not the (3*S*)-3,4-dihydroxy-2-methylidene-butanoate:

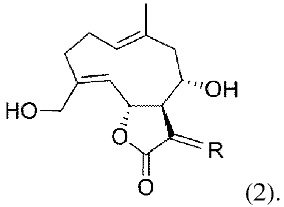

In preferred embodiments, the compound is the compound according to formula (3) as indicated below and pharmaceutically acceptable salts or esters thereof, with the proviso that the ester is not the (3S)-3,4-dihydroxy-2-methylidene-butanoate:

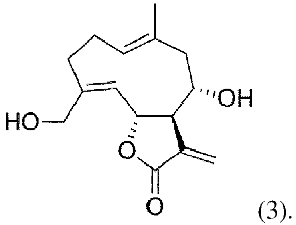

The compound of formula (3) usually is denoted salotenolide.

The compounds and particularly salotenolide can be usable in the form of solvates, hydrates, and pharmaceutically acceptable salts and esters. The term "pharmaceutically acceptable salt" refers to salts prepared from pharmaceutically acceptable non-toxic bases or acids. A pharmaceutically acceptable salt can be conveniently prepared from pharmaceutically acceptable non-toxic bases, including inorganic bases and organic bases, organic anions, organic cations, halides, or alkaline. The term pharmaceutically acceptable salt includes alkali metal salts and addition salts of free acids or free bases. Suitable pharmaceutically acceptable base addition salts include metallic salts and organic salts. Preferred salts derived from inorganic bases include ammonium, calcium, magnesium, potassium, and sodium salts. Salts derived from pharmaceutically acceptable organic, non-toxic bases include salts of primary, secondary, and tertiary amines. The compounds and particularly salotenolide, may be used in the form of a hydrochloride or maleate salt.

The compounds and particularly salotenolide, can be usable in the form of esters. The ester is, however, not the 3-(3,4-dihydroxy-2-methylidene-butanoate), which (3S)-3,4-dihydroxy-2-methylidene-butanoate of the compound of formula (3) is denoted cnicin. That is, cnicin is excluded from the compounds. The term "pharmaceutically acceptable ester" refers to esters of the hydroxy groups that are *in vivo* hydrolyzable. Examples of suitable *in vivo* hydrolyzable esters include those which break down readily in the human body to leave the parent compound or its salt. Pharmaceutically acceptable esters of the compounds are, in particular, physiologically readily hydrolyzable esters, for example, alkyl esters such as C₁₋₆ alkyl esters, phenyl, benzyl, or alkyl carboxylic acid esters or pivaloyloxymethyl, acetoxymethyl, phthalidyl, indanyl, and methoxymethylene esters. Preferred esters of the compounds and particularly of salotenolide, are methyl and ethyl esters.

As used herein, the term "axon" refers to a long, slender projection of a peripheral or central neuron. The axon typically conducts electrical impulses away from the neuron's cell body. The axon and its insulating sheath are called nerve fiber. In the central nervous system, myelin is produced by oligodendrocytes, while in the peripheral nervous system, it is formed by Schwann cells. A peripheral nerve fiber comprises an axon, myelin sheath, Schwann cells, and its endoneurium, a central nerve fiber does not comprise Schwann cells and endoneurium, but instead oligodendrocytes. As used herein, the term "axonal tip" refers to the terminal end or ends of an axon. At the tip of the axon is a dynamic compartment called the growth cone, via which growing axons move through their environment.

As used herein, the term "axonal damage" refers to damage to axons of any nerve fibers and nervous tissue. The term "nerve" as used herein refers to sensory fibers, motor fibers, autonomous fibers, or all. The term "axonal damage" refers to nerve injuries and peripheral neuropathies caused by axonal damage, and also to damage of the optic nerve or spinal cord or autonomous nervous system. Peripheral neuropathy can be the result of systemic diseases such as diabetes or leprosy, vitamin deficiency, medication, e.g., chemotherapy, radiation therapy, excessive alcohol consumption, immune system disease, or viral infection. Axonal damage may manifest upon nerve injury or disease. Nerve injury may be inflicted on nerves of the peripheral nervous system, for example, by a break or cutting off limbs, and also, the spinal cord may be injured by a cut, rupture, or compression/contusion. Axonal damage also may be associated with diseases inflicting injury on the axon, for example, axonal damage and axonal break caused by stroke or multiple sclerosis.

Nerve injury may be caused through a trauma such as laceration, focal contusion, stretch/traction injury, compression, drug injection injury, or electrical injury. Particular damage is a nerve cut, rupture, or compression/contusion. Nerve injuries, according to Seddon, are classified correlating the degree of injury with symptoms, pathology, and prognosis based on three main types of nerve fiber injury and whether there is continuity. Neurapraxia refers to a disorder of the peripheral nervous system in which the axon remains intact, but there is myelin damage causing an interruption in conduction of the impulse down the nerve fiber. Axonotmesis refers to a type of injury being the result of a more severe crush or contusion than neuropraxia, wherein both the nerve fibers and the nerve sheath are disrupted. Neurotmesis is the most severe lesion, which occurs on severe contusion, stretch, or laceration. Not only the axon, but the encapsulating connective tissue lose their continuity. The extreme degree of neurotmesis is transsection. In embodiments, nerve injury refers to axonotmesis or neurotmesis.

The axonal damage hence may be an injury of the peripheral nervous system or the autonomic nervous system or of the central nervous system that causes functional loss of the neuron. In embodiments, the axonal damage is an injury of the peripheral nervous system or the autonomic nervous system, particularly of the sciatic nerve, a cranial nerve, or the plexus brachialis. The sciatic nerve or nerves projecting into arms and fingers are long nerves and particularly vulnerable to damage. Damage can lead to irreversible loss of sensory and motor function as well as pain to patients. The axonal damage further may be an injury of a cranial nerve, particularly the trigeminal nerve, particularly its ophthalmic branch. The axonal damage also may be an injury of the plexus brachialis or the nerves that innervate internal organs.

In other embodiments, the axonal damage is an injury of the central nervous system, particularly of a central projection of the brain, the spinal cord or of the optic nerve. Axonal damage of the optic nerve or the spinal cord is an often occurring injury of the central nervous system. The optic nerve is the second of twelve paired cranial nerves. The optic nerve is considered to be part of the central nervous system (CNS) as it is derived from an outpouching of the diencephalon during embryonic development. Injury of the optic nerve often leads to lifelong and severe impairment of eyesight or even complete blindness of the respective eye. Axon regeneration usually does not occur in the injured spinal cord, which comprises other central projectories of axons. Axonal damage can lead to severe disabilities such as tetra- or paraplegia. For this reason, the axon growth-promoting effect of the compounds, particularly of salotenolide, may advantageously improve the clinical outcome after spinal cord injuries.

In further embodiments, the axonal damage is a damage of retinal ganglion cells, is associated with denervation of an injured or transplanted cornea, or is associated with axotomized fibers in the lesioned optic nerve or the trigeminal nerve, particular its ophthalmic branch. An injury of the cornea may be caused by cutting or abrasion. The cornea generally is the most densely innervated tissue on the surface of the body. Corneal transplantation, however, results in the cutting of corneal axons and thus incomplete denervation of the transplanted cornea. Re-innervation to the donor tissue is highly variable, and in many cases, hypoesthesia persists for many years after the initial surgery. Promotion of growth from axons arising from N. ophthalmicus into the cornea, particularly a transplanted cornea, thus is highly advantageous.

The axonal damage may be an axotomy in the central or peripheral nervous system. The axonal damage particularly may be an axotomy of the sciatic nerve, an axotomy of the optic nerve, an axotomy associated with axotomized fibers in a lesioned optic nerve, an axotomy associated with axotomized fibers in a lesioned trigeminal nerve, an axotomy associated with axotomized axons in an ophthalmic branch of a lesioned trigeminal nerve, an axotomy in the ophthalmic branch of the trigeminal nerve. The axonal damage also may be an axotomy in a cranial nerve or any spinal nerve.

A further impairment or disease that can be caused by axonal damage is glaucoma, in cases where axons are damaged in the optic nerve head. In other embodiments, the axonal damage or injury is associated with peripheral neuropathy, Charcot-Marie-Tooth disease, Guillain-Barré syndrome, glaucoma, stroke, or multiple sclerosis. Examples of peripheral neuropathies are diabetic neuropathy and cytostatics-induced neuropathy. Examples of axonal damage or injury in the CNS are stroke or multiple sclerosis.

In preferred embodiments, the compound is for use in promoting axonal regeneration. The term "axonal regeneration" refers to mechanisms where injured axons regenerate and also includes sprouting of uninjured axons from the cell body of the damaged neuronal cell, such that functional recovery can occur.

Particularly after peripheral nerve injury, axons can regenerate. While the distal portion of the axon, which is disconnected from the cell body, will undergo degeneration, proximal axons can regenerate and re-innervate their targets, allowing recovery of function.

The compound can be formulated as a pharmaceutical composition. A further aspect of the present invention relates to a pharmaceutical composition comprising as an active ingredient a compound according to general formula (1) as given as follows: 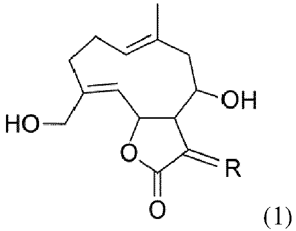 wherein:
- R: is selected from the group comprising -CH₂, -CHR', CR'₂, -NH and -NR",
- R': is selected from the group comprising C₁-C₁₀-alkyl, C₃-C₇-cycloalkyl, C₆-C₁₀-aryl, and C₇-C₁₀-arylalkyl; and
- R": is selected from the group comprising C₁-C₁₀-alkyl, C₃-C₇-cycloalkyl, C₆-C₁₀-aryl, and C₇-C₁₀-arylalkyl;
and/or racemates, enantiomers, diastereomers, solvates, hydrates, and pharmaceutically acceptable salts and/or esters thereof, with the proviso that the ester is not the 3-(3,4-dihydroxy-2-methylidene-butanoate), for use in the treatment of axonal damage.

In preferred embodiments, the compound is a compound according to formula (3) or a pharmaceutically acceptable salt or ester thereof. For the further description of the compound and the axonal damage, reference is made to the description above.

The pharmaceutical composition can comprise the compound as an active ingredient, a pharmaceutically acceptable carrier and optionally other therapeutic ingredients or adjuvants. The present invention hence also relates to a pharmaceutical composition for use in the treatment of axonal damage wherein the composition comprises as an active ingredient a compound for use according to the invention and a pharmaceutically acceptable carrier.

The compound can be dissolved or dispersed in a pharmaceutically acceptable carrier. The term "pharmaceutical or pharmacologically acceptable" refers to molecular entities and compositions that do not produce an adverse, allergic or other untoward reaction when administered to a subject, such as, for example, a human, as appropriate. The pharmaceutical carrier can be, for example, a solid, liquid, or gas. Suitable carriers and adjuvants can be solid or liquid and correspond to the substances ordinarily employed in formulation technology for pharmaceutical formulations. For example, water, glycols, oils, alcohols, and the like may be used to form liquid preparations such as solutions. Examples of solid carriers include lactose, terra alba, sucrose, talc, gelatin, agar, pectin, acacia, magnesium stearate, and stearic acid.

Examples of liquid carriers are sugar syrup, peanut oil, olive oil, and water. Examples of gaseous carriers include carbon dioxide and nitrogen.

The composition can be suitable for local, subcutaneous, oral or parenteral administration. In embodiments, the composition is formulated for local applications such as intraneural or periradicular application or systemic application such as intramuscular, intraperitoneal, intravenous, subcutaneous, intrathecal, or oral application. Parenteral administration includes subcutaneous, intramuscular, intravenous, intraneural, periradicular, intraperitoneal, and local administration. The composition may be formulated for local such as intraneural or periradicular application, or for systemic application such as intraperitoneal, intravenous, subcutaneous, or oral application. For the treatment of peripheral nerves, the composition may be administered systemically or intraneural, for example, into the sciatic nerve. Also, a local or topical application such as periradicular application may be preferred.

In further embodiments, the composition is formulated for intraocular application, particularly as eye drops. Intraocular administration, particularly in the form of eye drops, may be useful in the treatment of an injured or transplanted cornea or glaucoma, or axotomized fibers in the lesioned optic nerve or trigeminal nerve, particular its ophthalmic branch. Compositions suitable for injectable use include sterile aqueous solutions or dispersions.

For example, for treatment of the central nervous system, it may also be administered locally, by nerve injection, mini-pumps, etc. Still, it is preferred to administer the composition via an alimentary route. Specifically, the pharmaceutical composition may be administered systemically, for example, orally, or intraperitoneally. The pharmaceutical compositions may be conveniently presented in unit dosage form and prepared by any of the methods well known in the art of pharmacy. The pharmaceutical composition may be produced under sterile conditions using standard pharmaceutical techniques well known to those skilled in the art.

The present invention also relates to the use of a compound according to general formula (1) as given as follows: 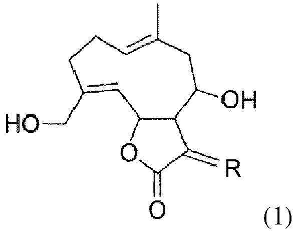 wherein:
- R: is selected from the group comprising -CH₂, -CHR', CR'₂, -NH and -NR",
- R': is selected from the group comprising C₁-C₁₀-alkyl, C₃-C₇-cycloalkyl, C₆-C₁₀-aryl, and C₇-C₁₀-arylalkyl; and
- R": is selected from the group comprising C₁-C₁₀-alkyl, C₃-C₇-cycloalkyl, C₆-C₁₀-aryl, and C₇-C₁₀-arylalkyl;
and/or racemates, enantiomers, diastereomers, solvates, hydrates, and pharmaceutically acceptable salts and/or esters thereof, with the proviso that the ester is not the 3-(3,4-dihydroxy-2-methylidene-butanoate), for the manufacture of a medicament for the treatment of axonal damage.

In preferred embodiments, the compound is a compound according to formula (3) or a pharmaceutically acceptable salt or ester thereof. For the further description of the compound and the axonal damage, reference is made to the description above.

The present invention also relates to a method of treating axonal damage, the method comprising administering to a subject a therapeutically effective amount of a compound according to general formula (1) as given as follows: 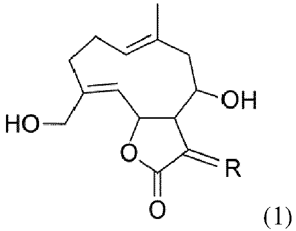 wherein:
- R: is selected from the group comprising -CH2, -CHR', CR'2, -NH and -NR",
- R': is selected from the group comprising C₁-C₁₀-alkyl, C₃-C₇-cycloalkyl, C₆-C₁₀-aryl, and C₇-C₁₀-arylalkyl; and
- R": is selected from the group comprising C₁-C₁₀-alkyl, C₃-C₇-cycloalkyl, C₆-C₁₀-aryl, and C₇-C₁₀-arylalkyl;
and/or racemates, enantiomers, diastereomers, solvates, hydrates, and pharmaceutically acceptable salts and/or esters thereof, with the proviso that the ester is not the 3-(3,4-dihydroxy-2-methylidene-butanoate).

The method preferably is a method of regenerating axonal damage. In preferred embodiments, the compound is a compound according to formula (3) or a pharmaceutically acceptable salt or ester thereof. For the further description of the compound and the axonal damage, reference is made to the description above.

Subjects include both human subjects and animal subjects, particularly mammalian subjects such as human subjects or mice or rats for medical purposes. The term "therapeutically effective amount" is used herein to mean an amount or dose sufficient to cause an improvement in a clinically significant condition in the subject. The treatment may be a continuous prolonged treatment or include a single or repetitive administration.

Unless otherwise defined, the technical and scientific terms used herein have the same meaning as commonly understood by one of ordinary skill in the art to which this invention belongs.

The examples which follow serve to illustrate the invention in more detail but do not constitute a limitation thereof.

The figures show:

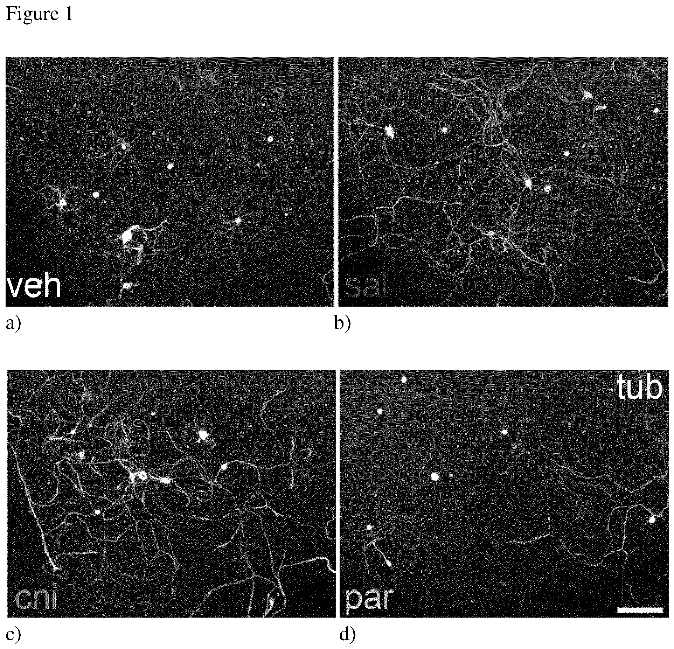

- Figure 1: in figures 1a), b) c) and d), respectively, dissociated dorsal root ganglion (DRG) neurons of mice treated with vehicle (veh), salotenolide (sal), cnicin (cni), or parthenolide (par) and stained for βIII-tubulin after 2 days in culture. Scale bar: 200 µm.

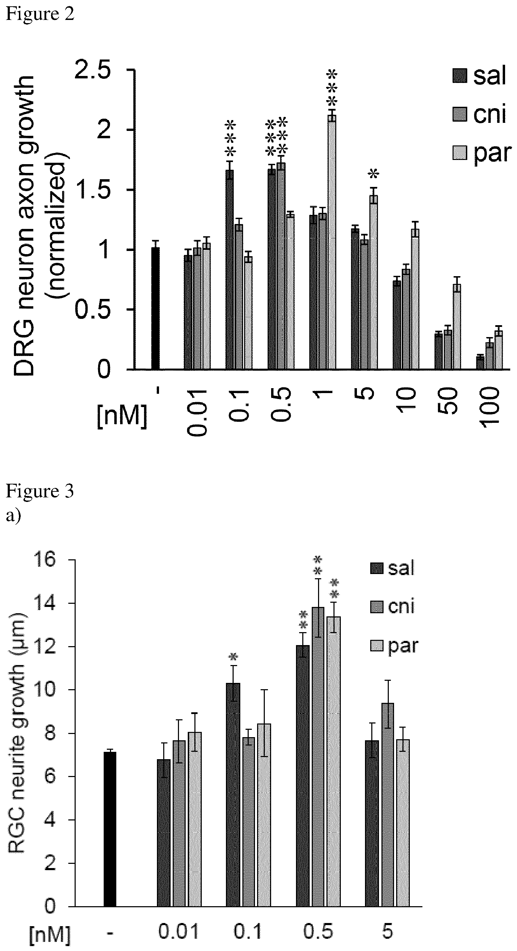

- Figure 2: the quantification of axon growth of DRG cultures after treatment with various salotenolide (sal), cnicin (cni), or parthenolide (par) concentrations. The error bars represent the standard error of the mean of two independent experiments. Statistical significance was determined with ANOVA and the post hoc Holm Sidac test. Treatment effects compared to vehicle-treated control group: *p ≤ 0.05 and ***p ≤ 0.001.

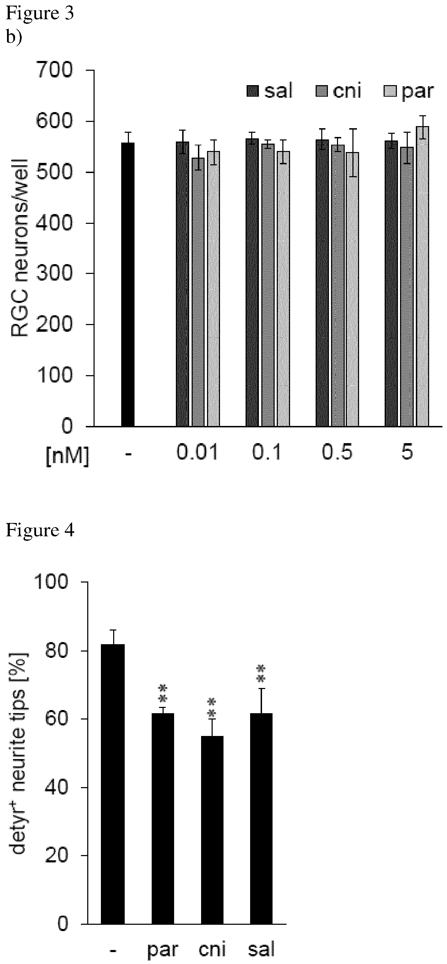

- Figure 3: in figure 3a) the quantification of neurite growth of adult retinal ganglion cell cultures after treatment with salotenolide (sal), cnicin (cni), or parthenolide (par) concentrations as indicated. Error bars represent mean ± SEM of two independent experiments. Treatment effects compared with vehicle control: *p ≤ 0.05; **p ≤ 0.01. Figure 3b) shows the quantification of RGC numbers in cultures after treatment with various concentrations of either salotenolide, cnicin, or parthenolide. Data represent the mean ± SEM of two independent experiments.
- Figure 4: the quantification of RGC neurite tips with detyrosinated tubulin in cultures treated with 0.5 nM salotenolide, cnicin, or parthenolide. Data represent the mean ± SEM of two independent experiments. Treatment effects compared with vehicle control: **p ≤ 0.01.

### Example 1: Determination of axonal regeneration in neuronal culture by salotenolide in vitro

The effect of the compound of formula (3), denoted salotenolide, on axon growth of mature neurons was determined in dissociated DRG neurons from adult wild type mice and compared to the effects of parthenolide and cnicin.

Dorsal root ganglion (DRG) neurons were isolated from adult wild type mice, as described in Gobrecht et al., Nature communications. 2014;5:4561. DRGs (T8-L6) were harvested, incubated in 0.25% trypsin/EDTA (GE Healthcare, Chalfont St Giles, UK) and 0.3% collagenase type IA (Sigma, St. Louis, US-MO) in DMEM (Life Technologies, Carlsbad, US-CA) at 37°C and 5% CO2 for 45 min and mechanically dissociated. Cells were resuspended in DMEM containing 10% fetal bovine serum (GE Healthcare) penicillin/streptomycin (500 U/ml; Merck Millipore, Billerica, US-MA) and 5-fluoro-2'-deoxyuridine (100 nM; Sigma). Cells were cultured on poly-D-lysine (PDL, 0.1 mg/ml, molecular weight < 300,000 kDa; Sigma) and laminin (20 µg/ml; Sigma) coated 96 well plates (Nunc, Germany) at 37°C and 5% CO₂.

Salotenolide, cnicin, and parthenolide (each Sigma-Aldrich) were dissolved in dimethyl sulfoxide (DMSO) to yield the appropriate concentrations. The cells were treated with vehicle, 0.01 nM, 0.1 nM, 0.5 nM, 1 nM, 5 nM, 10 nM, 50 nM or 100 nM of salotenolide, cnicin or parthenolide and cultured for 2 days. Afterward, cultures were stained with antibodies against βIII-tubulin (1:2,000; Covance).

The Figure 1 shows in figures 1a), b) c) and d), respectively, the DRG cells treated with vehicle, or 0.5 nM salotenolide (sal), cnicin (cni), or parthenolide (par). Figure 2 shows the quantification of axon growth of the neuronal cultures. Data from two independent experiments were normalized to the vehicle-treated control group with an average axon length of 594 µm/neuron.

As can be taken from figures 1 and 2, salotenolide promotes axonal damage and provided maximum effects already at a concentration of 0.1 nM and up to 5 nM. This shows that salotenolide provides a five times higher potency compared to cnicin and parthenolide, which means that a comparable effect on the promotion of axonal growth can be achieved by only about 20% of the concentration necessary of cnicin or parthenolide. Further, salotenolide provided significantly, about five times, a broader active concentration range compared to cnicin. As can be seen from figure 2, cnicin and parthenolide promote axonal regeneration only in a small concentration range of about 0.5-5 nM. In comparison, higher concentration ranges of 10 nM or more show no effects or even reduce axonal growth.

These results demonstrate that salotenolide promotes axonal regeneration with a higher potency compared to cnicin and parthenolide and provides a broader active concentration range compared to these compounds.

### Example 2: Determination of the effect of salotenolide on cells of the central nervous system

To test whether salotenolide is effective on cells of the central nervous system, neurite outgrowth of adult retinal ganglion cells (RCGs) was determined. To this end, adult murine retinae were dissociated and cultured for four days either in the presence of vehicle (-), or 0.01 nM, 0.1 nM, 0.5 nM, or 5 nM of salotenolide (sal), cnicin (cni), or parthenolide (par). Cells were fixed and stained for βIII tubulin, and neurite length per retinal ganglion cell was determined.

The quantification of neurite growth of the retinal ganglion cells after treatment with salotenolide (sal), cnicin (cni), or parthenolide (par) is shown in Figure 3. Figure 3 a) illustrates the quantification of neurite growth in retinal ganglion cell cultures after treatment with salotenolide (sal), cnicin (cni) or parthenolide (par) at concentrations of 0.01 nM, 0.1 nM, 0.5 nM, or 5 nM. Data represent the mean ± SEM of two independent experiments. Treatment effects were compared with vehicle control: *p ≤ 0.05; **p ≤ 0.01. Figure 3 b) illustrates the quantification of RGC numbers in cultures after treatment with salotenolide (sal), cnicin (cni) or parthenolide (par) at concentrations of 0.01 nM, 0.1 nM, 0.5 nM, or 5 nM. Data represent the mean ± SEM of two independent experiments.

As can be taken from Figure 3, salotenolide markedly, and significantly promoted neurite growth compared to untreated controls. Moreover, salotenolide provides a broader activity range.

These data demonstrate that salotenolide can promote nerve regeneration of central neurons. This finding suggests that salotenolide is usable to promote CNS regeneration, such as after injuries in the optic nerve or spinal cord.

### Example 3: Determination that salotenolide effects detyrosination of microtubules in vitro

To verify that the effect of salotenolide on neurite outgrowth of adult retinal ganglion cells (RCGs) was promoted via inhibition of microtubule detyrosination, axonal tips of cultured retinal ganglion cells were either exposed to vehicle (-) or 0.5 nM salotenolide (sal), cnicin (cni) or parthenolide (par). Three days after exposure, axons were stained for detyrosinated tubulin and βIII-tubulin.

Figure 4 shows the quantification of RGC neurite tips with detyrosinated tubulin in cultures treated with 0.5 nM salotenolide, cnicin or parthenolide. As can be taken from Figure 4, salotenolide treatment reduced levels of detyrosinated tubulin in axonal tips.

This indicates that the effects of salotenolide on axon growth is promoted via inhibition of microtubule detyrosination.

## Claims

1. A compound according to general formula (1) as given as follows: 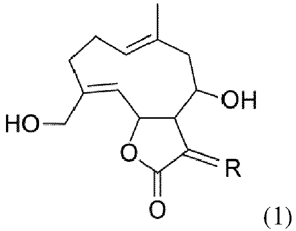 wherein:
R is selected from the group comprising -CH2, -CHR', CR'2, -NH and -NR",
R' is selected from the group comprising C₁-C₁₀-alkyl, C₃-C₇-cycloalkyl, C₆-C₁₀-aryl, and C₇-C₁₀-arylalkyl; and
R" is selected from the group comprising C₁-C₁₀-alkyl, C₃-C₇-cycloalkyl, C₆-C₁₀-aryl, and C₇-C₁₀-arylalkyl;
and/or racemates, enantiomers, diastereomers, solvates, hydrates, and pharmaceutically acceptable salts and/or esters thereof, with the proviso that the ester is not the 3-(3,4-dihydroxy-2-methylidene-butanoate), for use in the treatment of axonal damage.

2. The compound for use according to claim 1, wherein the compound is a compound according to formula (3) as indicated below and pharmaceutically acceptable salts or esters thereof, with the proviso that the ester is not the (3S)-3,4-dihydroxy-2-methylidene-butanoate:

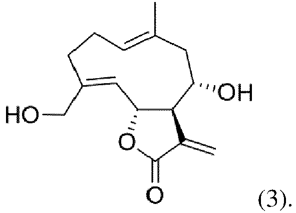

3. The compound for use according to claim 1 or 2, wherein the axonal damage is an injury of the peripheral nervous system or the autonomic nervous system, particularly of the sciatic nerve, a cranial nerve, or the plexus brachialis.

4. The compound for use according to any one of claims 1 to 3, wherein the axonal damage is an injury of the central nervous system, particularly of a central projection of the brain, the spinal cord or of the optic nerve.

5. The compound for use according to any one of claims 1 to 3, wherein the axonal damage is a damage of retinal ganglion cells, is associated with denervation of an injured or transplanted cornea, or is associated with axotomized fibers in the lesioned optic nerve or the trigeminal nerve, particular its ophthalmic branch.

6. The compound for use according to any one of claims 1 to 3, wherein the axonal damage is associated with peripheral neuropathy, Charcot-Marie-Tooth disease, Guillain-Barré syndrome, glaucoma, stroke, or multiple sclerosis.

7. The compound for use according to any one of claims 1 to 6, wherein the compound is for use in promoting axonal regeneration.

8. A pharmaceutical composition comprising as an active ingredient a compound according to general formula (1) as given as follows: 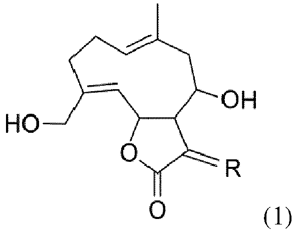 wherein:
R is selected from the group comprising -CH₂, -CHR', CR'₂, -NH and -NR",
R' is selected from the group comprising C₁-C₁₀-alkyl, C₃-C₇-cycloalkyl, C₆-C₁₀-aryl, and C₇-C₁₀-arylalkyl; and
R" is selected from the group comprising C₁-C₁₀-alkyl, C₃-C₇-cycloalkyl, C₆-C₁₀-aryl, and C₇-C₁₀-arylalkyl;
and/or racemates, enantiomers, diastereomers, solvates, hydrates, and pharmaceutically acceptable salts and/or esters thereof, with the proviso that the ester is not the 3-(3,4-dihydroxy-2-methylidene-butanoate), for use in the treatment of axonal damage.

9. The pharmaceutical composition according to claim 8, wherein the compound is a compound according to formula (3) and pharmaceutically acceptable salts or esters thereof, where 3,4-dihydroxy-2-methylidene-butanoate esters are excluded.

10. The pharmaceutical composition according to claim 8 or 9, wherein the composition is formulated for local application such as intraneural or periradicular application, or systemic application such as intramuscular, intraperitoneal, intravenous, subcutaneous, intrathecal or oral application.

11. The pharmaceutical composition according to claim 8 or 9, wherein the composition is formulated for intraocular application, particularly as eye drops.

12. Use of a compound according to general formula (1) as given as follows: 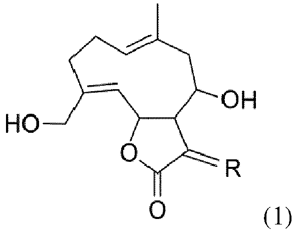 wherein:
R is selected from the group comprising -CH₂, -CHR', CR'₂, -NH and -NR",
R' is selected from the group comprising C₁-C₁₀-alkyl, C₃-C₇-cycloalkyl, C₆-C₁₀-aryl, and C₇-C₁₀-arylalkyl; and
R" is selected from the group comprising C₁-C₁₀-alkyl, C₃-C₇-cycloalkyl, C₆-C₁₀-aryl, and C₇-C₁₀-arylalkyl;
and/or racemates, enantiomers, diastereomers, solvates, hydrates, and pharmaceutically acceptable salts and/or esters thereof, with the proviso that the ester is not the 3-(3,4-dihydroxy-2-methylidene-butanoate), for the manufacture of a medicament for the treatment of axonal damage.

13. A method of treating axonal damage, the method comprising administering to a subject a therapeutically effective amount of a compound according to general formula (1) as given as follows: 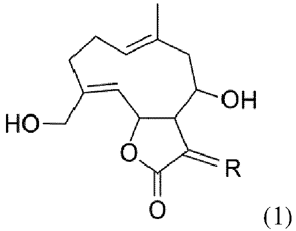 wherein:
R is selected from the group comprising -CH₂, -CHR', CR'₂, -NH and -NR",
R' is selected from the group comprising C₁-C₁₀-alkyl, C₃-C₇-cycloalkyl, C₆-C₁₀-aryl, and C₇-C₁₀-arylalkyl; and
R" is selected from the group comprising C₁-C₁₀-alkyl, C₃-C₇-cycloalkyl, C₆-C₁₀-aryl, and C₇-C₁₀-arylalkyl;
and/or racemates, enantiomers, diastereomers, solvates, hydrates, and pharmaceutically acceptable salts and/or esters thereof, with the proviso that the ester is not the 3-(3,4-dihydroxy-2-methylidene-butanoate).
